# EUROPÄISCHE PATENTANMELDUNG

(11) **EP 1 967 230 A1**
(43) Veröffentlichungstag der Anmeldung: **10.09.2008**
(21) Anmeldenummer: 08101199.1
(22) Anmeldetag: 01.02.2008
(51) Int. Cl.: A61N 5/10

(54) **Partikeltherapie-Anlage**

(30) Priorität: 08.03.2007 DE 102007011399
(71) Anmelder: SIEMENS AKTIENGESELLSCHAFT, 80333 München (DE)
(72) Erfinder: Kaiser, Werner, 91052, Erlangen (DE); Use, Tim, 90469, Nürnberg (DE)

(57) **Zusammenfassung**

Die Erfindung betrifft eine Partikeltherapieanlage umfassend
- einen Behandlungsraum,
- eine drehbare Gantry mit einer Bestrahlungseinheit, über die in dem Behandlungsraum ein Partikelstrahl aus verschiedenen Winkeln abgegeben werden kann,
- einen bewegbaren Boden im Behandlungsraum,
- eine Bildgebungsvorrichtung, die auf dem beweglich gelagerten Boden angeordnet ist,

wobei der bewegbare Boden derart ausgebildet ist, dass er ein Gewicht der Bildgebungsvorrichtung tragen kann.

## Beschreibung

Die Erfindung betrifft eine Partikeltherapieanlage umfassend eine drehbare Gantry mit einer Bestrahlungseinheit, über die in einem Behandlungsraum ein Partikelstrahl aus verschiedenen Winkeln abgegeben werden kann, wobei in dem Behandlungsraum eine Bildgebungsvorrichtung angeordnet ist.

Bei einer Partikeltherapie, insbesondere von Krebserkrankungen, wird in einem geeigneten Beschleuniger ein Partikelstrahl beispielsweise aus Protonen oder Schwerionen erzeugt. Der Partikelstrahl wird in einem Strahlungskanal geführt und tritt über ein Austrittsfenster des Strahlungskanals in einen Behandlungsraum ein. In vielen Fällen ist wegen der aufwändigen Strahlungsführung lediglich ein ortsfestes Strahlaustrittsfenster vorgesehen. Bei einigen Anlagen ist jedoch eine drehbare Gantry mit einem Austrittsfenster vorgesehen. Wegen der aufwändigen Strahlungsführung ist die Gantry sehr großvolumig aufgebaut. Die Gantry umschließt einen oftmals zylinderförmigen Behandlungsraum, in den ein Patiententisch hineingefahren wird. Für eine möglichst präzise Behandlung muss das zu bestrahlende Gewebe des Patienten möglichst genau im Isozentrum (der Treffpunkt des Strahles bei der Rotation der Gantry) der Anlage positioniert werden.

Am Ende des Strahlungskanals sind in einer auch als Nozzle bezeichnet Bestrahlungseinheit unmittelbar vor dem Austrittsfenster üblicherweise mindestens ein Strahlendetektor sowie passive Strahlelemente angeordnet. Um den Patienten auch von unten bestrahlen zu können, ist die Gantry idealerweise um 360° um den Patienten rotierbar. Hierbei besteht das Problem, dass die Bestrahlungseinheit auch in dem Bereich unterhalb des Patienten rotierbar sein muss. Für diesen Zweck ist der Boden eines Behandlungsraums mit einer Gantry üblicherweise beweglich ausgebildet.

Damit der Partikelstrahl möglichst präzise den zu behandelnden Bereich des Patienten trifft, ist eine genaue Ausrichtung des Patienten relativ zu dem Partikelstrahl notwendig. Um diese genaue Ausrichtung zu gewährleisten und auch überprüfen zu können, wird üblicherweise eine Bildgebungsvorrichtung im Behandlungsraum eingesetzt, mit der die Position des Patienten bzw. des zu bestrahlenden Bereichs überprüft werden kann. Eine derartige Bildgebungsvorrichtung umfasst üblicherweise eine Röntgenquelle und einen Röntgendetektor, mit dem Röntgenbilder des zu behandelnden Patienten aufgezeichnet werden können. Es können aber auch andere Bildgebungssysteme eingesetzt werden, wie beispielsweise ein Positronen-Emissions-Tomograph.

Insbesondere bei Behandlungsräumen mit einer drehbar gelagerten Gantry stellt die Anordnung der Bildgebungsvorrichtung aufgrund von Platzproblemen und aufgrund von vielen beweglichen Elementen, die mit der Bildgebungsvorrichtung kollidieren können, eine Herausforderung dar.

In der DE 10 2004 048 212 A1 ist beispielsweise eine Strahlentherapieanlage mit einer Bildgebungsvorrichtung offenbart, bei der seitlich an einem Therapiestrahlauslass jeweils ein Röntgendetektor angeordnet ist, denen jeweils ein Röntgenstrahler gegenüberliegt. Eine Positionierung der Röntgensysteme erfolgt durch Drehung der gesamten Gantry. Eine flexibel einzusetzende Bildgebung ist hierdurch nur eingeschränkt möglich.

Es ist demzufolge die Aufgabe der Erfindung, eine Partikeltherapieanlage mit einem Behandlungsraum mit einer drehbar gelagerten Gantry anzugeben, bei dem eine Bildgebungsvorrichtung in einer Weise angeordnet ist, die gleichzeitig eine flexible Bildgebung weitgehend unabhängig von der Stellung der Gantry ermöglicht.

Die Aufgabe wird erfindungsgemäß gelöst durch eine Partikeltherapieanlage nach Anspruch 1.

Die Partikeltherapieanlage umfasst demnach:
- einen Behandlungsraum,
- eine drehbare Gantry mit einer Bestrahlungseinheit, über die in dem Behandlungsraum ein Partikelstrahl aus verschiedenen Winkeln abgegeben werden kann,
- einen bewegbaren Boden im Behandlungsraum, und
- eine Bildgebungsvorrichtung, die auf dem beweglich gelagerten Boden angeordnet ist.

Der bewegbare Boden ist dabei so stabil ausgebildet, dass er die Bildgebungsvorrichtung, die auf dem Boden angeordnet ist, stabil tragen kann. Auf diese Weise ist es möglich, die Bildgebungsvorrichtung Platz sparend im Behandlungsraum anzuordnen. Die Gantry kann dabei um eine Drehachse drehbar gelagert werden. Üblicherweise umschließt die Gantry den Behandlungsraum.

Die Bildgebungsvorrichtung ist dabei fest auf dem beweglich gelagerten Boden angeordnet. Im Gegensatz zu Bildgebungsvorrichtungen, die zur Bildgebung in den Behandlungsraum und auf den Boden gefahren werden und anschließend wieder entfernt werden, ermöglicht die hier offenbarte Bildgebungsvorrichtung eine sofortige Verfügbarkeit, da sie im Behandlungsraum verbleibt, und beispielsweise nur ein C-Bogen an die passende Stelle zur Bildgebung verfahren werden muss.

In einer Ausführungsform weist der bewegbare Boden zumindest ein Bodensegment auf, auf dem die Bildgebungsvorrichtung angeordnet ist. In vorteilhafter Weise verbleibt das Bodensegment bei jeder Stellung der Gantry innerhalb des Behandlungsraumes. Hierdurch kann die Bildgebungsvorrichtung unabhängig von der Stellung der Gantry zur Bildgebung verwendet werden. Ein aufwändiges Verfahren der Bildgebungsvorrichtung entfällt.

Das Bodensegment kann als einstückiges Element insbesondere mit einem einfachen Aufbau ausgebildet sein. Auf diese Weise kann die mechanische Belastbarkeit und die notwendige Tragfähigkeit des Bodensegments auf einfache Weise erreicht werden. Die Bildgebungsvorrichtung kann dann auf dem einen Bodensegment angeordnet sein.

In vorteilhafter Weise ist das Bodensegment bei jeder Stellung der Gantry von der Gantry selbst umschlossen. Hierdurch befindet sich die Bildgebungsvorrichtung stets innerhalb der Gantry, wodurch eine Nähe der Bildgebungsvorrichtung zum Patienten bei jeder Stellung der Gantry gegeben ist.

In einer Ausführungsform ist der bewegbare Boden auf der Gantry beweglich gelagert. Hierdurch ist es möglich, dass das Gewicht der Bildgebungsvorrichtung und/oder das Gewicht des Bodens im Wesentlichen durch die Gantry selbst oder über die Gantry abgestützt werden. Im Vergleich zu Bodenelementen, die keine Abstützung direkt unterhalb des Bodenelements aufweisen und beispielsweise seitlich über weitere verbundene Bodensegmente getragen wird, ergeben sich auf diese Weise Vorteile bezüglich der mechanischen Konstruktion. Beispielsweise kann der bewegbare Boden auf einer Seitenwand der Gantry gelagert sein. Die Seitenwand der Gantry kann sich unterhalb des bewegbaren Bodens erstrecken, sodass sich der Boden nun auf der Seitenwand abstützt.

In vorteilhafter Weise weist der Boden eine Geometrie auf, die der Gantry angepasst ist. Hierdurch kann eine Bewegung der Gantry und/oder des Bodens auf besonders einfache Weise ausgeführt werden, ohne dass eine komplizierte Abstimmung der Bewegungen zueinander erfolgen muss.

In vorteilhafter Weise umfasst die Bildgebungsvorrichtung eine Haltevorrichtung und eine Bildgebungseinheit. Die Bildgebungseinheit kann durch die Haltevorrichtung in verschiedenen Positionen positioniert werden. Durch diese Ausführungsform kann eine variable Positionierung der Bildgebungseinheit im Raum auf einfache Weise erreicht werden. Hierdurch ist es möglich, die Bildgebungseinheit in verschiedenen Positionen zur Bildgebung einzusetzen, was die Flexibilität erhöht. Die Bildgebungseinheit selbst kann dabei beispielsweise ein C-Bogen sein, an dessen Enden eine Röntgenquelle bzw. ein Röntgendetektor angebracht ist.

In einer vorteilhaften Ausführungsform ist die Haltevorrichtung für die Bildgebungseinheit als Roboterarm ausgebildet, der insbesondere mehrere Gelenke aufweist. Hierdurch ist eine besonders flexible Positionierung der Bildgebungseinheit möglich.

In vorteilhafter Weise ist es möglich, die positionierbare Bildgebungseinheit in einer Parkstellung so zu positionieren, dass sie an einer Rückwand der Gantry angeordnet ist. Auf diese Weise kann die Bildgebungseinheit in einer Position geparkt werden, in der sie weitgehend vor Kollisionen geschützt ist.

Ausführungsformen der Erfindung mit vorteilhaften Weiterbildungen gemäß den Merkmalen der abhängigen Ansprüche werden in der folgenden Zeichnung näher erläutert, ohne jedoch darauf beschränkt zu sein.

Es zeigen:
- Figur 1: eine Vorderansicht auf einen zylinderförmigen Behandlungsraum einer Partikeltherapieanlage mit einer Bildgebungseinheit,
- Figur 2: einen Längsschnitt durch den zylinderförmigen Behandlungsraum gemäß Figur 1, und
- Figur 3: einen schematischen Überblick über eine Partikeltherapieanlage.

Gleiche Bezugszeichen haben in den Figuren die gleiche Bedeutung.

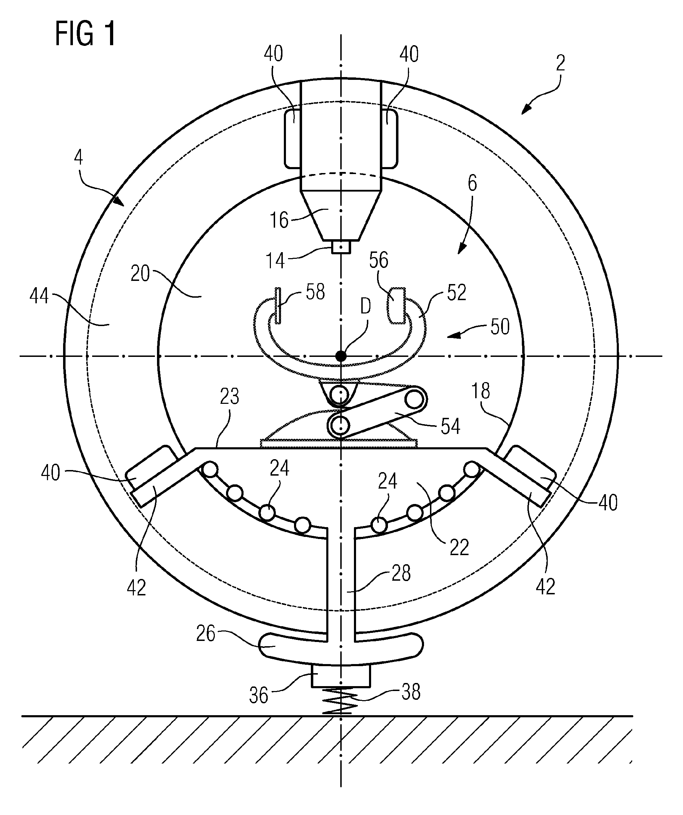

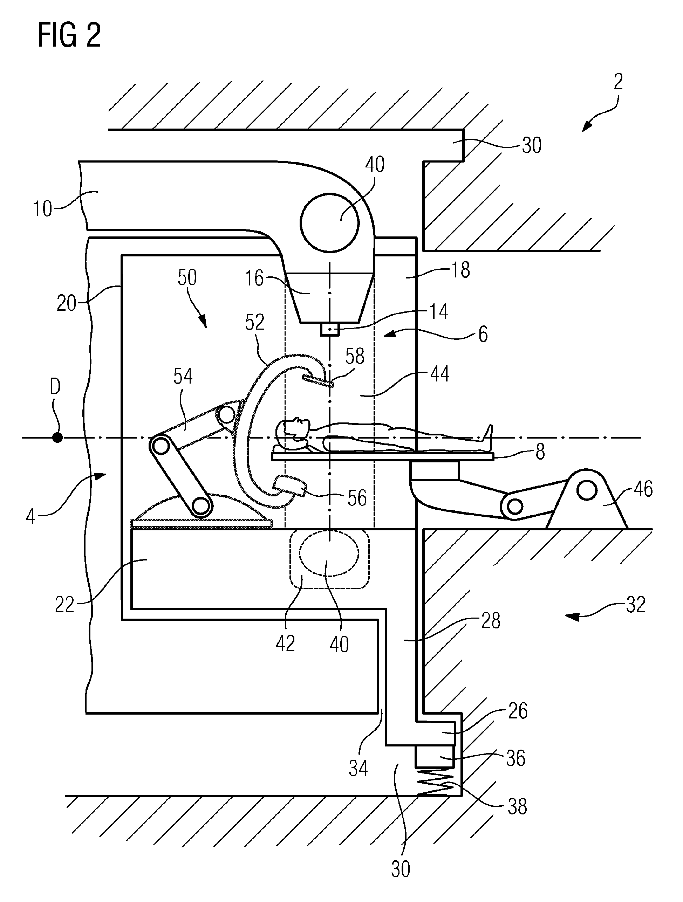

In Figur 1 und Figur 2 ist aus verschiedenen Perspektiven ein Behandlungsraum 6 einer Partikeltherapie-Anlage 2 mit einer um eine Drehachse D drehbaren Gantry 4 dargestellt. Die Drehachse D verläuft bei der Darstellung der Partikeltherapie-Anlage gemäß Figur 1 senkrecht zur Zeichnungsebene und ist deshalb durch einen Punkt D angedeutet. Die Gantry 4 umschließt den etwa zylinderförmigen Behandlungsraum 6, in dem ein Patiententisch 8 positionierbar ist, wie aus Figur 2 zu entnehmen ist. Die Gantry 4 umfasst außerdem einen teilweise gezeigten Strahlungskanal 10. Im Strahlungskanal 10 wird ein hier nicht dargestellter Partikelstrahl, beispielsweise ein Schwerionen- oder Protonenstrahl zur Behandlung eines auf dem Patiententisch 8 liegenden Patienten 12 geführt. Der Partikelstrahl tritt über ein Austrittsfenster 14 einer Bestrahlungseinheit 16 in den Behandlungsraum 6 ein, wobei die Bestrahlungseinheit 16 aus einer rotierbaren, tragfähigen Seitenwand 18 des Behandlungsraums 6 herausragt. Nach hinten ist der Behandlungsraum 6 durch eine Rückwand 20 begrenzt, die in diesem Ausführungsbeispiel eine einfache Verkleidung ohne mechanische Belastbarkeit ist und mit der Gantry 4 um die Drehachse D rotiert.

Der Behandlungsraum 6 weist weiterhin einen Boden auf, der aus einem einzigen massiven kreisbogenförmigen Bodensegment 22 besteht, das auf der Seitenwand 18 der Gantry 4 gelagert ist. Das Bodensegment 22 hat im gezeigten Ausführungsbeispiel eine waagerechte Nullposition angenommen und bildet somit eine begehbare Bodenfläche 23.

Auf dieser Bodenfläche 23 ist eine Bildgebungsvorrichtung 50 angeordnet. Die Bildgebungsvorrichtung 50 umfasst eine Bildgebungseinheit 52, die hier als C-Bogen ausgebildet ist, an dessen Enden ein Röntgenstrahler 56 bzw. ein Röntgendetektor 58 angeordnet sind. Über eine Haltevorrichtung 54, die hier als Roboterarm mit mehreren Gelenken ausgebildet ist, kann die Bildgebungseinheit 52 im Raum positioniert werden. Mit der Bildgebungseinheit 52 wird die Position des Patienten 8 vor und/oder während der Behandlung überprüft. Die Bodenfläche kann aber auch durch eine Wand oder einen Deckenkonstruktion erweitert sein, die Bildgebungsvorrichtung 50 kann entsprechend mit diesen Konstruktionen verbunden werden.

Die Bildgebungseinheit 52 kann dabei in einer Parkstellung positioniert werden, wie es in Figur 1 gezeigt ist. In dieser Stellung befindet sich die Bildgebungseinheit 52 an der Rückwand 20 der Gantry 4. Hierdurch behindert die Bildgebungsvorrichtung 50 weder die Positionierung des Patienten 8 noch die Bestrahlung des Patienten 8 durch den Partikelstrahl. Zur Bildgebung kann die Bildgebungseinheit 52 aus der Parkstellung heraus verfahren werden, und an die gewünschte Position um den Patienten 8 herum positioniert werden. Eine mögliche Position während des Betriebs der Bildgebungsvorrichtung 50 ist in Figur 2 gezeigt.

Die Tragfähigkeit des Bodensegments 22 ist den Erfordernissen entsprechend gewählt, so dass sowohl die Bildgebungsvorrichtung 50 als auch etwaiges Bedienpersonal (hier nicht dargestellt) tragen kann.

Das Bodensegment 22 ist über eine Anzahl von Lagern 24, die in diesem Ausführungsbeispiel Rollenlager sind, derart auf der Seitenwand 18 gelagert, dass das Bodensegment 22 und die Seitenwand 18 zueinander relativ beweglich sind. Dies bedeutet, dass die Gantry 4 rotieren kann, wobei das Bodensegment 22 in seiner Nullposition bleibt. Alternativ kann das Bodensegment 22 angetrieben von einer äußeren Kraft über seine Rollenlager 24 bei feststehender Gantry 4 eine Bewegung um die Drehachse D ausführen. Möglich ist auch, dass zum Verfahren des Bodensegments 22 um die Drehachse D seine Lage gegenüber der Gantry 4 fest bleibt und es bei der Rotation der Gantry mitgeführt wird.

Das Bodensegment 22 weist einen Ausleger 26 auf, der über einen sich senkrecht zu der horizontalen Bodenfläche 23 erstreckenden Steg 28 mit dem Bodensegment 22 verbunden ist. Zur Führung des Auslegers 26 und des Steges 28 beim Verfahren des Bodensegments 22 um die Drehachse D sind eine um den Behandlungsraum 6 umlaufende Kulisse 30, die insbesondere in einem den Behandlungsraum 6 angrenzenden Festboden 32 ausgebildet ist bzw. ein zwischen der Gantry 4 und dem Festboden 32 gebildeter Schacht 34 vorgesehen. Darüber hinaus ist zum Festhalten des Bodensegments 22 in der Nullposition ein Reibelement 36 vorgesehen, das an dem Ausleger 26 angreift und über eine Feder 28 elastisch gelagert ist.

Die in Figur 1 und Figur 2 gezeigte Anordnung hat den Vorteil, dass bei sehr vielen Bestrahlungswinkeln ein ortsfester Boden im Behandlungsraum 6 vorhanden ist. Z.B. ist ausgehend von der gezeigten senkrechten Lage der Bestrahlungseinheit 16 oberhalb des Bodensegments 22 eine Rotation der Bestrahlungseinheit 16 um 90° im Uhrzeigersinn sowie entgegen dem Uhrzeigersinn möglich, ohne dass dabei eine Kollision mit dem Bodensegment 22 droht. In diesem Winkelbereich muss also das Bodensegment 22 nicht weggefahren werden. Erst bei einer Auslenkung der Bestrahlungseinheit 16 aus der gezeigten Position um einen Drehwinkel, der größer ist als +/- 90°, erfolgt ein Verschieben des Bodensegments 22 um die Drehachse D. Hierfür kann das Bodensegment 22 einen eigenen Antrieb aufweisen, so dass angesteuert von einer Steuereinheit das Bodensegment 22 um die Drehachse D verfahren wird, noch bevor ein Kontakt zwischen dem Bodensegment 22 und der Bestrahlungseinheit 16 entsteht. In dem gezeigten Ausführungsbeispiel ist allerdings kein separater Antrieb bzw. keine separate Ansteuerung des Bodensegments 22 vorgesehen, sondern es erfolgt eine Zwangsführung des Bodensegments 22 über die Bestrahlungseinheit 16, die bei ihrer Drehung das Bodensegment 22 vor sich schiebt.

Falls das Bodensegment 22 um die Drehachse D bewegt wird, bewegt sich die Bildgebungsvorrichtung 50 entsprechend mit. Um das Risiko von möglichen Kollisionen der Bildgebungsvorrichtung 50 mit anderen Elementen zu vermeiden, kann die Bildgebungsvorrichtung 50 während der Bewegung in die Parkposition verfahren werden. In der ausgelenkten Position des Bodensegmentes kann nach Stillstand der Gantry dann wieder eine Bildaufnahme mit der Bildgebungsvorrichtung 50 erfolgen. Dafür ist die Bestimmung des Winkels des Bodensegments 22 gegen die Nulllage über einen geeigneten Winkelaufnehmer wesentlich.

Um einen harten Stoß zwischen der Bestrahlungseinheit 16 und dem Bodensegment 22 zu vermeiden, sind sowohl an der Bestrahlungseinheit 16 als auch am Bodensegment 22 im Bereich einer Kontaktstelle zwischen den beiden Pufferelemente 40 angeordnet. Damit ein möglichst großflächiger Kontakt zwischen dem Bodensegment 22 und der Bestrahlungseinheit 16 erfolgt, weist das Bodensegment 22 beidseitig zwei geneigte Flügel 42 auf, die sich außerhalb des Behandlungsraums 6 ausstrecken und die Pufferelemente 40 des Bodensegments 22 tragen. Durch die geneigte Stellung der Flügel 42 wird insbesondere ein günstiger Winkel eingestellt, so dass die Pufferelemente 40 des Bodensegments 22 und der Bestrahlungseinheit 16 großflächig aufeinander aufliegen, wenn die Bestrahlungseinheit 16 bei ihrer Drehung das Bodensegment 22 erreicht. Um die Flügel 42 außerhalb des Behandlungsraums anordnen zu können, ist in der Seitenwand 18 eine umlaufende Vertiefung 44 vorgesehen, die in den Figuren durch eine gestrichelte Linie angedeutet ist.

Das Bodensegment 22 ist in diesem Ausführungsbeispiel nach Art einer Leichtmetallkonstruktion ausgebildet, so dass der Antrieb der Gantry 4 beim Verschieben des Bodensegments 22 durch die Bestrahlungseinheit 16 möglichst wenig ausgelastet wird. Bei einer Auslenkung der Bestrahlungseinheit 16 ausgehend von ihrer in den Figuren gezeigten Vertikallage um einen Auslenkwinkel, der größer als 180° ist, summieren sich die Komponenten der Gewichtskraft sowohl des Bodensegments 22 als auch der Bestrahlungseinheit 16 die in Richtung zurück zur Nullposition wirken, was eine sehr hohe Belastung für den Antrieb der Gantry 4 bedeutet. Damit der Antrieb nicht unnötig belastet wird, ist bei der Partikeltherapie-Anlage 2 vorgesehen, dass die Bestrahlungseinheit 16 ausgehend von ihrer Vertikallage nur bis 180° im Uhrzeigersinn oder entgegen dem Uhrzeigersinn verfahren wird, wodurch allerdings der gesamte Winkelbereich von 360° abgedeckt ist.

Der Patiententisch 8 wird in diesem Ausführungsbeispiel über einen Roboterarm 46 in den Behandlungsraum 6 hineingefahren. Dabei hat der Patiententisch 8 keinen Kontakt mit dem Bodensegment 22. Der Roboterarm 46 ist hierbei ein mehrachsiger Industrieroboterarm mit einer mehrteiligen Mechanik und ist auf dem Festboden 32 montiert. Mit Hilfe des Roboterarms 46 wird der Patiententisch 8 translatorisch in die horizontale sowie in die vertikale Richtung bewegt. Hierbei ist ein Drehen des Roboterarms 46 um mehrere Achsen möglich, so dass die Bewegung des Patiententisches 8 sich durch drei translatorische Freiheitsgrade sowie drei Rotationsfreiheitsgrade auszeichnet. Durch die Translations- und Rotationsbewegung des Patiententisches 8 werden die Lage und die Entfernung des Patienten 12 zur Bestrahlungseinheit 16 eingestellt. Während seiner Positionierung in dem Behandlungsraum 6 bleibt der Patiententisch 8 in einer horizontalen Lage, damit der Patient 12 stabil liegt.

Der hier beschriebene Behandlungsraum 6 der Partikeltherapie-Anlage 2 zeichnet sich dadurch aus, dass eine Bestrahlung des Patienten 12 von allen Winkellagen möglich ist, wobei ein problemloses Verfahren der Bestrahlungseinheit 16 unterhalb des Patiententisches 8 gewährt ist, indem das Bodensegment 22 über seine bewegliche Lagerung von der Bestrahlungseinheit 16 selbst weggeschoben wird. Diese Ausgestaltung hat den wesentlichen Vorteil, dass kein zusätzlicher Antrieb sowie keine separate Ansteuerung des Bodensegments 22 erforderlich sind. Zudem kann das Bodensegment 22 für sehr viele Bestrahlungswinkel in seiner Nullposition bleiben, wodurch ein begehbarer und befahrbarer Behandlungsraumboden auch während der Behandlung des Patienten 12 vorhanden ist.

Zudem ist durch die Anordnung der Bildgebungsvorrichtung 50 auf dem bewegbaren Boden 22 eine vorteilhafte Positionierung der Bildgebungsvorrichtung 50 im Behandlungsraum 6 gegeben. Dadurch, dass das Bodensegment 22 unabhängig von der Stellung der Gantry 4 stets im Behandlungsraum 6 verbleibt, ist die Bildgebungsvorrichtung 50 stets für eine Bildgebung verfügbar. Das Gewicht der Bildgebungsvorrichtung 50 ist über das Bodensegment 22 und über die Gantry 4 abgestützt, so dass aufwändige Halterungskonstruktionen für eine Bildgebungsvorrichtung entfallen.

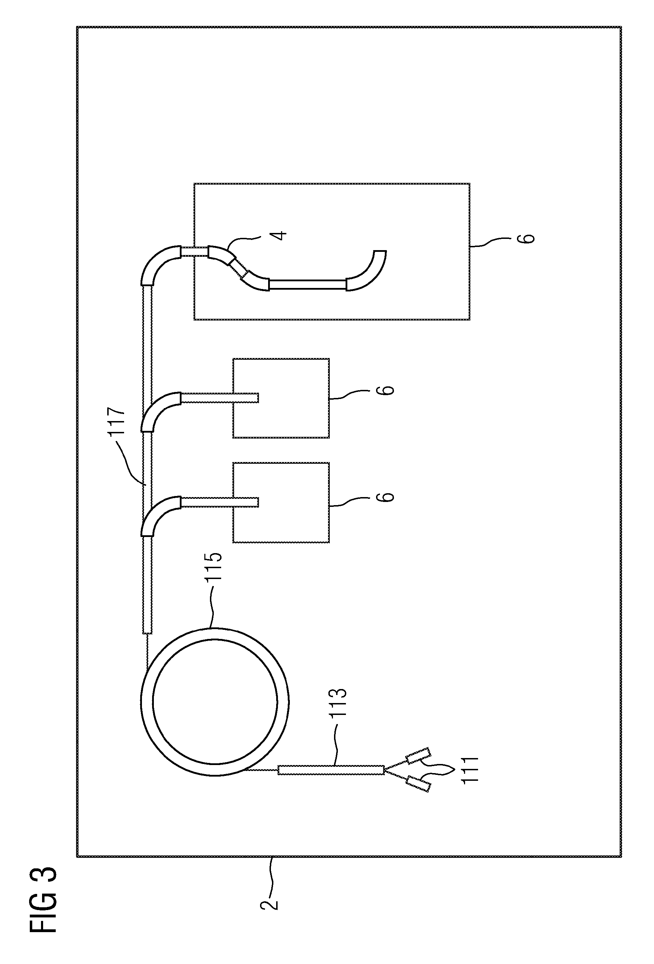

Figur 3 zeigt einen schematischen Überblick über den Aufbau einer Partikeltherapieanlage 2. In einer Partikeltherapieanlage 2 erfolgt die Bestrahlung insbesondere von tumorerkranktem Gewebe mit einem Partikelstrahl. Als Partikel werden vornehmlich Ionen wie beispielsweise Protonen, Pionen, Heliumionen, Kohlenstoffionen oder andere Ionensorten eingesetzt.

Üblicherweise werden derartige Partikel in einer Partikelquelle 111 erzeugt und in einem Vorbeschleuniger 113, z.B. einem linearen Beschleuniger (LINAC für engl.:"LINear ACcelerator"), beschleunigt. Anschließend werden die Partikel in einen Beschleuniger 115, beispielsweise ein Synchrotron oder Zyklotron, eingespeist, in dem sie auf hohe Energien, wie sie zur Bestrahlung nötig sind, beschleunigt werden. Nachdem die Partikel den Beschleuniger 115 verlassen, führt ein Hochenergiestrahl-Transportsystem 117 den Partikelstrahl zu den gewünschten Bestrahlungsräumen 6. In einem Bestrahlungsraum 6 werden die beschleunigten Partikel auf einen Patienten gerichtet. Je nach Ausgestaltung erfolgt dies von einer festen Richtung (im so genannten "fixed beam"-Räumen) aus oder aber über eine bewegliche rotierbare Gantry 4 von verschiedenen Richtungen aus. Bei den letztgenannten Behandlungsräumen 6 kommt die Anordnung der Bildgebungsvorrichtung auf einem bewegbaren Boden, wie sie beispielsweise vorstehend anhand von Figur 1 und Figur 2 beschrieben ist, zur Anwendung.

Dieser Grundaufbau einer Partikeltherapieanlage 2 ist typisch für viele Partikeltherapieanlagen, kann aber auch hiervon abweichen.

## Patentansprüche

1. Partikeltherapieanlage umfassend
- einen Behandlungsraum,
- eine drehbare Gantry mit einer Bestrahlungseinheit, über die in dem Behandlungsraum ein Partikelstrahl aus verschiedenen Winkeln abgegeben werden kann,
- einen bewegbaren Boden im Behandlungsraum,
- eine Bildgebungsvorrichtung, die auf dem beweglich gelagerten Boden angeordnet ist,
wobei der bewegbare Boden derart ausgebildet ist, dass er ein Gewicht der Bildgebungsvorrichtung tragen kann.

2. Partikeltherapieanlage nach Anspruch 1,
**dadurch gekennzeichnet ,**
**dass** der bewegbare Boden zumindest ein Bodensegment aufweist, auf dem die Bildgebungsvorrichtung angeordnet ist.

3. Partikeltherapieanlage nach Anspruch 1,
**dadurch gekennzeichnet ,**
**dass** der bewegbare Boden als einstückiges Bodensegment ausgebildet ist, auf dem die Bildgebungsvorrichtung angeordnet ist.

4. Partikeltherapieanlage nach Anspruch 3,
**dadurch gekennzeichnet ,**
**dass** das Bodensegment bei jeder Stellung der Gantry innerhalb des Behandlungsraumes verbleibt.

5. Partikeltherapieanlage nach Anspruch 3 oder 4,
**dadurch gekennzeichnet ,**
**dass** sich das Bodensegment bei jeder Stellung der Gantry von der Gantry umschlossen ist.

6. Partikeltherapieanlage nach einem der Ansprüche 1 bis 5,
**dadurch gekennzeichnet ,**
**dass** der bewegbare Boden beweglich auf der Gantry gelagert ist.

7. Partikeltherapieanlage nach einem der Ansprüche 1 bis 6,
**dadurch gekennzeichnet ,**
**dass** der Boden eine an die Gantry angepasste Geometrie aufweist.

8. Partikeltherapieanlage nach einem der Ansprüche 1 bis 7,
**dadurch gekennzeichnet ,**
**dass** die Bildgebungsvorrichtung eine Haltevorrichtung und eine Bildgebungseinheit umfasst, wobei die Bildgebungseinheit durch die Haltevorrichtung positionierbar ist.

9. Partikeltherapieanlage nach einem Anspruch 8,
**dadurch gekennzeichnet ,**
**dass** die Haltevorrichtung als Roboterarm ausgebildet ist und insbesondere mehrere Gelenke aufweist.

10. Partikeltherapieanlage nach Anspruch 8 oder 9,
**dadurch gekennzeichnet ,**
**dass** die positionierbare Bildgebungseinheit in einer Parkstellung an einer Rückwand der Gantry positionierbar ist.
